(19) Europäisches Patentamt / European Patent Office / Office européen des brevets

(11) **EP 2 737 055 B1**

(12) **EUROPEAN PATENT SPECIFICATION**

(45) Date of publication and mention of the grant of the patent:
**11.11.2020 Bulletin 2020/46**

(21) Application number: **12740650.2**

(22) Date of filing: **23.07.2012**

(51) Int Cl.:
*A61K 35/36* (2015.01)    *C12N 5/071* (2010.01)

(86) International application number:
**PCT/GB2012/051759**

(87) International publication number:
**WO 2013/014435 (31.01.2013 Gazette 2013/05)**

(54) **MICRO ORGAN COMPRISING MESENCHYMAL AND EPITHELIAL CELLS**

MIKRO ORGAN, DAS MESENCHYMALE UND EPITHELIALE ZELLEN ENTHÄLT

MICRO ORGANE CONTENANT DES CELLULES MESENCHYMATEUSES ET EPITHELIALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.07.2011 GB 201112922**

(43) Date of publication of application:
**04.06.2014 Bulletin 2014/23**

(73) Proprietor: **University Of Durham**
**Old Elvet**
**Durham DH1 3HP (GB)**

(72) Inventors:
• **GUO, Aihua**
  **Gilesgate**
  **Durham DH1 2LB (GB)**
• **JAHODA, Colin Albert Buchanan**
  **Richmond**
  **Yorkshire DL10 7AU (GB)**

(74) Representative: **HGF**
**1 City Walk**
**Leeds LS11 9DX (GB)**

(56) References cited:
**EP-A1- 1 734 113      EP-A1- 2 034 011**
**WO-A2-03/055990      WO-A2-2007/100870**
**US-A- 5 755 814**

• YEN C M ET AL: "High-throughput reconstitution of epithelialmesenchymal interaction in folliculoid microtissues by biomaterial-facilitated self-assembly of dissociated heterotypic adult cells", BIOMATERIALS, ELSEVIER SCIENCE PUBLISHERS BV., BARKING, GB, vol. 31, no. 15, 1 May 2010 (2010-05-01), pages 4341-4352, XP026971511, ISSN: 0142-9612 [retrieved on 2010-03-05]
• HIGGINS ET AL: "Modelling the hair follicle dermal papilla using spheroid cell cultures", EXPERIMENTAL DERMATOLOGY ONLINE, WILEY-BLACKWELL PUBLISHING LTD, vol. 19, no. 6, 1 June 2010 (2010-06-01), pages 546-548, XP002585390, ISSN: 1600-0625, DOI: 10.1111/J.1600-0625.2009.01007.X [retrieved on 2010-04-20]
• HIGGINS ET AL: "Modelling the hair follicle dermal papilla using spheroid cell cultures", EXPERIMENTAL DERMATOLOGY ONLINE, WILEY-BLACKWELL PUBLISHING LTD, US, vol. 19, no. 6, 1 June 2010 (2010-06-01), pages 546-548, XP002585390, ISSN: 1600-0625, DOI: 10.1111/J.1600-0625.2009.01007.X [retrieved on 2010-04-20]

Remarks:
The file contains technical information submitted after the application was filed and not included in this specification

## Description

This invention is directed to a micro-organ comprised of epithelial and mesenchymal cells and exemplified by a micro-skin equivalent composed of skin epidermal and dermal cells, and a novel model for investigation of skin activities and responses.

## Background

The development and maintenance of a large number of organs and structures in the mammalian body involves key epithelial-mesenchymal interactions. Amongst these are lungs, testes, ovaries, kidneys, prostate and mammary and salivary glands. Epithelial-mesenchymal interactions also underpin the development and growth of integumental structures including teeth, and skin and skin appendages such as hair follicles. They are also central to the development and maintenance of specific parts of more complex structures such as the cornea of the eye.

Human adult skin provides a physical and chemical barrier to protect the host against invasion by toxins and microorganisms and prevent dehydration that can result from loss of barrier function. When skin integrity is lost, wound healing, including scar formation, is crucial in order to restore this barrier. The loss of skin integrity and function due to wound injury has led to efforts designed to better comprehend the molecular and cellular mechanisms that can optimize wound repair.

Autologous grafting of skin from other parts of the patient is the current surgical norm. Often, however, such as in whole body burn cases, the amount of skin available is limited. In addition, extra skin damage is created at the donor site. The outcome for patients with widespread epidermal damage mainly depends on the localized capacity of the surviving epidermal keratinocytes to divide and proliferate. Culturing epidermis enables the grafting of epidermal keratinocytes that have preserved sufficient proliferative capacity. Whilst used clinically, there are drawbacks with the current method of culturing cells. The very thin and fragile nature of cultured epidermis limits its usage when deep skin damage is involved. In this regard, bioengineered in vitro 3D tissues known as human skin equivalents have been intensively investigated to offer a favourable material for wound healing.

Skin substitutes for pharmaceutical research and clinical application have been widely studied. Since Medawar (Medawar, PB. Sheets of pure epidermal epithelium from human skin. Nature (Lond) 148: 783-4 (1941)) successfully separated a pure epidermal sheet from human skin by trypsinisation, it has been possible to obtain epidermal cells for tissue culture and, subsequently, for bioengineered skin substitutes which have emerged over the past 20 years as the most carefully studied and proven of the advanced wound management technologies (Metcalfe AD, Ferguson MW. Biomaterials. 2007 Dec;28(34):5100-13).

The complexity of skin has to some extent been bioengineered in in vitro 3D tissues known as human skin equivalents (HSE) that have many morphologic and phenotypic properties of human skin. Some skin equivalents are currently commercially available. However, such commercial products are often expensive, and laboratory culturing requires complicated techniques, considerable manpower and long periods of time. Furthermore, the involvement of a variation of scaffolds to inoculate skin cells such as a collagen gel from animal products, polymer mesh, nylon net or a human acellular dermal matrix requires that such materials be optimized for their ultimate use when implanted into patients.

For example, Berg et al. disclose a skin model system used as in vitro test system or for therapeutic purposes, comprising 3-D, cross-linked matrix of insoluble collagen containing fibroblasts and stratified layers of differentiated epidermal cells supported thereon (U.S. Patent 5,888,248 and U.S. Patent 5,945,101). The method employed for producing such a system comprises seeding a 3-D, cross-linked collagen matrix with fibroblasts and culturing the seeded matrix under conditions to allow in-growth and proliferation of the fibroblasts, and then seeding the surface of the matrix with epidermal cells in a manner to deter in-growth of the epidermal cells. The seeded matrix is cultured to first allow the epidermal cells to attach to the matrix and proliferate to form a monolayer, and then to allow the epidermal cells to differentiate.

Building skin equivalents on a collagen matrix, or sponge, is also disclosed by Eisenberg in WO 91/16010. Hewitt et al. also disclose the incorporation of further components, such as blood plasma and thrombin, within a fibrin three-dimensional matrix on which to construct a living tissue equivalent (WO 03/041568).

There are more than 2,000 known skin disorders. Many are very common and well-understood, and many still do not have a cure. Since the success in integrating transgenic technology into the study of the pathogenesis of skin diseases, hundreds of characterized mouse strains are now available for skin disease research. However, with the increasing difficulties associated with in vivo animal studies and the ethical and cost restraints of human studies in vitro, the need for in vitro models of skin, including disease models, is growing for purposes of both clinical studies and the regulatory assessment of drugs and chemicals from topical formulations.

Clinically therefore, and for in vitro studies, there exists a need for organ equivalents or organ models armed solely with good quality living mesenchymal and epithelial cells, for example, a skin equivalent or model comprising dermal and epidermal cells.

Yen et al describe high-throughput reconstitution of epithelial-mesenchymal interaction in folliculoid microtissues by biomaterial-facilitated self-assembly of dissociated heterotypic adult cells (C M Yen, C C Chan, S J Lin, Biomaterials, Volume 31, Issue 15, 2010, pages 4341-4352).

EP 2 034 011 describes a cell cluster comprising plural kinds of cells derived from soma with ability to form primitive organ like structures.

EP 1 734 113 describes a method for obtaining a skin equivalent.

WO 2007/100870 describes methods for compact aggregation of dermal cells.

## Summary of the Invention

In a first aspect the invention provides a micro-organ composite which comprises a core group of cells and an outer layer of cells, wherein the cells of the core group are mesenchymal cells and the cells of the outer layer are epithelial cells, wherein the core group of cells is fully encapsulated by the outer layer of cells.

In a second aspect the invention provides a micro-organ composite which comprises a core group of cells and an outer layer of cells, wherein the cells of the core group are epithelial cells and the cells of the outer layer are mesenchymal cells, wherein the core group of cells is

fully encapsulated by the outer layer of cells.

Preferably, the mesenchymal cells and/or the epithelial cells are derived from more than one cell source.

Preferably, the mesenchymal cells are dermal cells, and wherein the epithelial cells are epidermal cells, still more preferably said epidermal cells are keratinocytes Preferably, the composite is essentially free of an added biomaterial.

Preferably, said mesenchymal core and epithelial outer layer interact to form a basement membrane.

The core group of cells is fully encapsulated by the outer layer of cells.

Preferably, the composite is in the form of a spherical particulate, more preferably, wherein the spherical particulate has a size between 40 and 500 microns.

In a third aspect the invention provides a composite in accordance with the preceding aspects for use as a medicament.

In a fourth aspect the invention a composite in accordance with the preceding aspects for use in skin wound healing. Preferably, the dermal cells are dermal fibroblasts and the epithelial cells are keratinocytes.

In a fifth aspect the invention provides a composite in accordance with the preceding aspects for use for use in treating alopecia. Preferably, the dermal cells are follicular dermal cells and the epithelial cells are outer root sheath keratinocytes.

In a sixth aspect the invention provides a pharmaceutical composition comprising a composite accordance with the preceding aspects together with a pharmaceutically acceptable carrier.

In preferred embodiments said composites or compositions are prepared for topical administration.

In a seventh aspect the invention provides a method of producing a micro-organ cell composite, comprising: a) growing disaggregated mesenchymal cells in a hanging drop culture to form an aggregate core of cells; and b) adding epithelial cells to the aggregate core of cells, wherein the epithelial cells grow to form an outer layer on the aggregate core of cells.

In an eighth aspect the invention provides a method of producing a micro-organ cell composite, comprising: a) growing disaggregated epithelial cells in a hanging drop culture to form an aggregate core of cells; and b) adding mesenchymal cells to the aggregate core of cells, wherein the mesenchymal cells grow to form an outer layer on the aggregate core of cells.

Preferably the mesenchymal cells are dermal cells, and wherein the epithelial cells are epidermal cells, more preferably said epidermal cells are keratinocytes.

Preferably the aggregate core of cells and the outer layer establish a basement membrane, preferably in 10 days or less, 9 days or less, 8 days or less, 7 days or less, 6 days or less, 5 days or less, 4 days or less, 3 days or less or 2 days or less.

Preferably, the cells of the composite are viable for about 2 weeks or more, or about 3 weeks or more.

In a ninth aspect the invention provides an *in vitro* model for studying a skin disease or disorder comprising a composite in accordance with the preceding aspects.

In a tenth aspect the invention provides use of a composite osite in accordance with the preceding aspects as an *in vitro* skin model.

Preferably, said skin model is a skin disease or disorder model.

In an eleventh aspect the invention provides a method of screening an agent for the treatment of a skin disease or disorder comprising: a) providing a composite a composite in accordance with the preceding aspects; b) exposing said composite to an agent; and c) determining whether the agent has a therapeutic effect on the composite.

Preferably the skin disease or disorder is a keloid, a tumour, a wound, psoriasis, eczema or dermatitis.

In a twelfth aspect the invention provides a method of screening for any molecular or chemical agent: a) providing a composite in accordance with the preceding aspects; b) exposing said composite to an agent; and c) determining whether the agent has a toxic effect on the composite, or affects cell growth or viability, or alters gene expression in the composite cells.

## Brief Description of the Drawings

Embodiments of the invention are further described hereinafter with reference to the accompanying drawings, in which:

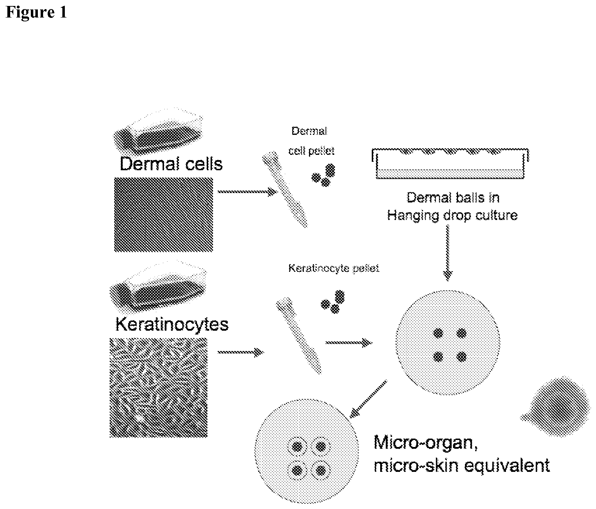

Figure 1 depicts a basic method of micro-organ formation. Core aggregates are first established with one cell type, in this case the skin dermal cells, and then these are surrounded by keratinocytes.

Figure 2 depicts immunofluorescence images of sectioned micro-organs. DF = dermal fibroblasts; DP = dermal papilla cells; K = keratinocytes. The basal layer of the outer epithelium/epidermis stains with the stem type marker p63. Dermal and epidermal cells express characteristic markers fibronectin and involucrin, respectively.

Figure 3 depicts immunofluorescence images of sectioned micro-organs. DS = dermal sheath cells; DFi = dermal fibroblasts; DP = dermal papilla cells; K = keratinocytes. The basal layer of the outer epithelium stains with the stem type marker p63. Dermal cells express characteristic markers fibronectin and vimentin.

Figure 4 depicts immunofluorescence images of sectioned micro-organs. DS = dermal sheath cells; DF = dermal fibroblasts; DP = dermal papilla cells; K = keratinocytes. The dermal sheath cells in particular have secreted large amounts of extracellular material which is seen as amorphous pools of labelled material inside the structures.

Figure 5 depicts examples of cultured dermal cells migrating from 7 day-old micro-skin equivalents. This shows that micro-skin equivalent dermal cells grow well when returned to culture.

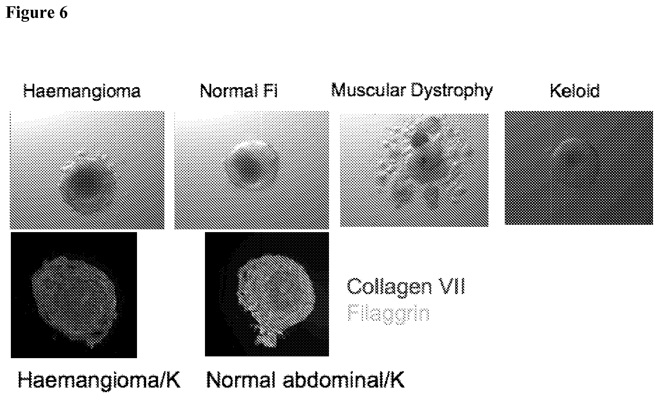

Figure 6 shows how micro-skin equivalents can be made with HaCaT keratinocytes and fibroblasts from disease patients. The top line shows external views of micro-organs created by combining fibroblasts from normal patients and from patients with different diseases (i.e. haemangioma, muscular dystrophy, and keloid) with HaCaT cells, an epidermal cell line. Not all dermal cells are equally competent; cells from muscular dystrophy patients fail to produce proper double layer structures. The lower line shows immunofluorescence images of sectioned micro-organs with dermal cells from both normal and haemangioma skin expressing type VII collagen, and epidermal cells expressing the differentiation marker filaggrin.

Figure 7 illustrates the relationship between dermal cell number and sphere diameter in micro-skin cell composites.

Figure 8 depicts the variation between predicted and observed sizes of micro-skin cell composites.

Figure 9 depicts the aggregation of epidermal cells in 1:1 KGM2:MEM media with different foetal calf serum concentrations.

Figure 10 illustrates how micro-organ cell composite formation is an active process

**Detailed Description of the Invention**

As embodied and broadly described herein, the present invention is directed to micro-organs comprising mesenchymal and epithelial cells, methods of forming such micro-organs, and uses for such micro-organs.

The present inventors have advantageously identified that hanging drop co-culture of mesenchymal and epithelial cells, wherein said cells are introduced at staggered intervals of the co-culture, produces three dimensional micro-organ cell composites, in which the mesenchymal and epithelial cells interact in a physiological manner to provide a basement membrane there between. This micro-organ cell composite provides a versatile system for producing *in vitro* organ models, in particular, for producing three dimensional skin models. Advantageously, the micro-organ cell composites of the invention are particularly suited to automated set-up and high though put screening.

As used herein, the term "micro-organ cell composite", refers to an isolated artificial cell structure, i.e. not naturally occurring in the human or animal body, cell composites. The micro-organ cell composites of the invention provide an *ex vivo* organ structure that closely represents *in vivo* organ structure.

Micro-organ cell composites

The invention provides a micro-organ cell composite comprising a core group of cells and an outer layer of cells, wherein the cells of the core group are mesenchymal cells and the cells of the outer layer are epithelial cells, and wherein the core group of cells is fully encapsulated by the outer layer of cells. Alternatively, the invention provides a micro-organ cell composite comprising a core group of cells and an outer layer of cells, wherein the cells of the core group are epithelial cells and the cells of the outer layer are mesenchymal cells, and wherein the core group of cells is fully encapsulated by the outer layer of cells.

As used herein, the phrase "at least partially encapsulated" requires that an outer surface the mesenchymal core of the micro-organ cell composite is at least partially surrounded by epithelial cells. The core is fully encapsulated by the outer layer of cells.

The body comprises many different epithelial cells. In one embodiment an epithelial cell or one or more epithelial cells in accordance with the invention may be a simple epithelium, such as simple squamous epithelium, such as mesothelium or endothelium. Alternatively, an epithelial cells may be a stratified epithelia, such as an epidermal cell or columnar epithelia cell. Such cells may include epithelial cells of the eye cornea. Said epidermal cells may be differentiated epidermal cells or epidermal progenitor cells. By epidermal progenitor cell is meant a multipotent cell having epidermal potential, e.g. a cell capable of differentiating into an epidermal cell.

In a preferred embodiment the epithelial cells are keratinocytes, preferably epidermal keratinocytes or a corneal keratinocytes.

Preferably, the epithelial cells are mammalian cells, more preferably human epithelial cells. The epithelial cells can be freshly isolated cells or multiple passaged cells.

The body comprises many different mesenchymal cells. In one embodiment a mesenchymal cell or at least one or more mesenchymal cells in accordance with the invention include fibroblasts, adipocytes, chondroblasts, osteoblasts and stromal cells from different regions of the body including the bone marrow, prostate, heart, lung, blood vessels and tendons. Preferably, the mesenchymal cells are dermal cells, fibroblasts or histocytes. Preferably, the mesenchymal cells are interfollicular dermal cells or hair follicle dermal cells. More preferably, the dermal cells are dermal fibroblasts.

In addition to skin epidermal cells from normal interfollicular skin, micro-organ cell composites were made using adult and neonatal epidermal cells purchased from commercial suppliers and the immortal HaCaT cell line.

Preferably, the mesenchymal cells are mammalian cells, more preferably human mesenchymal cells. The mesenchymal cells can be freshly isolated cells or multiple passaged cells.

In one embodiment the mesenchymal and/or epithelial cells are cells isolated from disease tissues.

The mesenchymal cells and/or the epithelial cells may comprise more than one type of mesenchymal and/or epithelial cell.

In the micro-organ cell composite of the invention, the mesenchymal cells, i.e. dermis, remain functional provides the necessary support for epithelial, i.e. epidermal proliferation and differentiation. Accordingly, the micro-organ composite of the invention permits the reciprocal mesenchymal/epithelial interactions found in organ, i.e. skin development and maintenance.

Preferably, the micro-organ cell composite is a micro-skin cell composite, wherein said mesenchymal cells are dermal cells and the epithelial cells are epidermal cells, more specifically, where the dermal cells are dermal fibroblasts and the epithelial cells are keratinocytes. The inventors have surprisingly demonstrated that in such a micro-skin cell composite the dermal/epidermal interactions are such that the cells organize themselves as *in vivo* into the two essential skin layers, without any added biomaterials. Moreover, the inventors have demonstrated basement membrane formation between the mesenchymal core and the epithelial outer layer. Formation of the basement membrane demonstrates interaction between the mesenchymal core and epithelial outer layer, since each cell type contributes to its formation. Specifically, two basement membrane components that are seen both in native skin and in the micro-skin cell composite of the invention are type IV and type VII collagen. The epithelial cells of a micro-skin cell composite of the inventions, i.e., keratinocytes, possess a specific gene profile: keratin-10 are transcribed by keratinocytes from the stratum spinosum and involucrin; filaggrin are transcribed in the stratum lucidum and expressed in the stratus corneum. P63 is regarded as a marker of epidermal stem cells which are located in the basal cells above the basement membrane. A well-formed basement membrane is identified by laminin and collagen VII. Functional dermal markers such as vimentin and fibronectin are expressed by the dermal cell aggregates.

Preferably, a micro-skin cell composite has the following features: a) the epithelial and mesenchymal cell types have no added biomaterials and are interacting; b) epithelial cells are capable of proliferation and express differentiation markers while mesenchymal cells show typical markers; and c) basement membrane constituents are present in between the epithelial and mesenchymal layers.

Alternatively, the micro-organ cell composite is a micro-follicular cell composite, wherein said mesenchymal cells are follicular dermal cells and the epithelial cells are epidermal cells, more specifically, where the dermal cells are follicular dermal cells and the epithelial cells are outer root sheath keratinocytes. In this regard, epithelial-mesenchymal interactions are also crucial for normal skin hair follicle development, which is initiated by reciprocal crosstalk between an epidermal placode and a dermal condensation. Indeed, hair follicle morphogenesis is driven by epithelium-mesenchymal interactions through different stages of development and through adult hair cycles.

The micro-organs disclosed herein can also be stored frozen and returned to culture, allowing for easy transportation and commercialization.

Preferably, the micro-organ cell composites are in the form of a spherical particulate.

The size of the micro-organ cell particulate is dependent upon the number of cells forming the mesenchymal core. Preferably, the spherical micro-organ composite has a diameter of from 40 to 500 $\mu$m, more preferably from 60 to 300 $\mu$m.

Advantageously, the artificial micro-organ cell composite remains viable in vitro and ex vivo.

Formation of Micro-organ cell composites

The invention provides a method of producing a micro-organ cell composite, comprising:

a) growing disaggregated mesenchymal cells in a hanging drop culture to form an aggregate core of cells; and

b) adding epithelial cells to the aggregate core of cells,

wherein the epithelial cells grow to form an outer layer on an outer surface of the aggregate core of cells.

In addition, the invention provides a method of producing a micro-organ cell composite, comprising:

a) growing disaggregated epithelial cells in a hanging drop culture to form an aggregate core of cells; and
b) adding mesenchymal cells to the aggregate core of cells,

wherein the mesenchymal cells grow to form an outer layer on an outer surface of the aggregate core of cells.

A hanging drop culture is a culture technique in which a material to be cultivated, i.e. cells, is inoculated into a culture medium, i.e. a cell culture medium, and drops of the inoculated culture medium are placed onto a culture surface, i.e. a glass slide, a petri dish, a cover glass etc., and the culture surface then inverted. As a result of gravity the material to be cultivated, i.e. cells, aggregates at the apex of the drop of culture medium. The aggregate is prevented from penetrating the surface of the drop as a result of surface tension.

As used herein, the term "aggregate" refers to the aggregation of cells into a cluster, more specifically to the aggregation of mesenchymal cells, preferably into a single cell type population cluster. The cell aggregate is formed prior to addition of the cells which grow to form the outerlayer.

Preferably, said mesenchymal cells are dermal cells and the epithelial cells are epidermal cells, more specifically, where the dermal cells are dermal fibroblasts and the epithelial cells are keratinocytes.

Preferably, disaggregated mesenchymal cells, i.e. dermal cells form aggregates when grown in hanging drop cultures. Skin epidermal cells are then added to the dermal structures. The epidermal cells grow around the dermal cells, and the two cell layers interact with each other and preferably establish a basement membrane. Both cell layers have tissue specific protein expression and behaviour typical of their activities in skin.

Preferably, the disaggregated epithelial cells are cultured for 48 to 72 hours to form an aggregate core of cells, prior to addition of the mesenchymal cells. Preferably, the epithelial aggregate is cultured with the mesenchymal cells for approximately 30 to 72 hours, preferably 36 hours, such that the mesenchymal cells grow to form an outer layer on an outer surface of the aggregate core.

Alternatively, the disaggregated mesenchymal cells are cultured for 48 to 72 hours to form an aggregate core of cells, prior to addition of the epithelial cells. Preferably, the mesenchymal aggregate is cultured with the epithelial cells for approximately 20 and 72 hours, preferably 24 hours, such that the epithelial cells grow to form an outer layer on an outer surface of the aggregate core.

In a preferred embodiment of the invention, the method comprises growing disaggregated dermal cells in a hanging drop culture to form an aggregate core of cells; and adding epithelial cells, preferably epidermal cells, more preferably keratinocytes to the aggregate core of cells, wherein the micro-organ cell composite is formed in approximately 50 hours to 7 days of hanging drop culture, or in approximately 60 hours to 7 days of hanging drop culture, preferably in 4 to 6 days, and more preferably 3 or less days. This compares favourably with a conventional "skin equivalent" model which requires a collagenous substrate, involves many more cells, and takes 3-4 weeks to constitute. Gangatirkar, P., et al. Establishment of 3D organotypic cultures using human neonatal epidermal cells. Nat. Protoc. 2: 178-86 (2007).

Cell culture media useful in the hanging drop culture in the present invention includes any composition capable of supporting the growth of a mesenchymal and epithelial cell composite.

Media which can be employed in accordance with the hanging drop culture of the invention usually comprise one or more carbon sources, nitrogen sources, inorganic salts, vitamins and/or trace elements. Such suitable cell culture media are well known in the art and include, by way of example only Minimum Essential Medium Eagle, Minimum Essential Medium Dulbecco, ADC-I, LPM (Bovine Serum Albumin-free), FIO(HAM), F12 (HAM), DCCM1, DCCM2, RPMI 1640, BGJ Medium (with and without Fitton-Jackson Modification), Basal Medium Eagle (BME-with the addition of Earle's salt base), Dulbecco's Modified Eagle Medium (DMEM- without serum), Yamane, IMEM-20, Glasgow Modification Eagle Medium (GMEM), Leibovitz L-15 Medium, McCoy's 5A Medium, Medium M199 (M199E-with Earle's sale base), Medium M 199 (M 199H- with Hank's salt base), Minimum Essential Medium Eagle (MEM-E-with Earle's salt base), Minimum Essential Medium Eagle (MEM-H-with Hank's salt base) and Minimum Essential Medium Eagle (MEM-NAA with non essential amino acids), among numerous others, including medium 199, CMRL 1415, CMRL 1969, CMRL 1066, NCTC 135, MB 75261, MAB 8713, DM 145, Williams' E, Williams' G, Neuman & Tytell, Higuchi, MCDB 301, MCDB 202, MCDB 501, MCDB 401, MCDB 411 and MDBC 153.

A preferred medium for use in the present invention is the commercially available media, MEM Eagle.

In one embodiment the hanging drop culture medium is preferably a serum free culture medium. Alternatively, the hanging drop culture medium is supplemented with serum, such as bovine foetal serum. In a further embodiment the hanging drop culture medium may be supplemented with growth factors, cytokines, and hormones, for example growth hormone, erythropoeitin, thrombopoietin, interleukin 3, interleukin 6, interleukin 7, macrophage colony stimulating factor, c-kit ligand/stem cell factor, osteoprotegerin ligand, insulin, insulin like growth factors, epidermal growth factor, fibroblast growth factor, nerve growth factor, cilary neurotrophic factor, platelet derived growth factor, and bone morphogenetic protein.

In a further embodiment the hanging drop culture medium may be further supplemented with antibiotics, albumin, amino acids, or other components known in the art for the culture of cells.

Preferably, all hanging drop culture media components are sterilized, either by heat or by filter sterilization. The components may be sterilized either together or, if required, separately.

Preferably, the hanging drop culture is performed at a temperature from 15°C to 45°C, preferably from 25°C to 40°C, more preferably from 25 to 37 °C, more preferably from 32 to 37°C, more preferably at 37°C, and may be kept constant or may be altered during culture.

The methods disclosed herein require a minimal number of cells, a shorter length of time for formation, less manpower and technical expertise, and are reproducible and relatively economic. Cells remain viable for a relatively long period when compared to conventional skin models (e.g. 14 days or more, preferably 3 weeks or more) and are capable of faithfully reflecting their character *in vivo.*

Uses

The inventors have surprisingly identified that micro-organ cell composites of the invention have the ability to produce outgrowth when returned to two-dimensional monolayer culture, or when returned to a natural substrate (e.g. the cornea stroma or a wound bed). Accordingly, the micro-organ cell composites of the invention are of particular use in various therapeutic settings.

Accordingly, the invention provides a pharmaceutical composition comprising a micro-organ cell composite in accordance with the invention together with a pharmaceutically acceptable excipient, diluent or carrier.

Preferably, the micro-organ cell composite is a micro-skin cell composite, wherein said mesenchymal cells are dermal cells and the epithelial cells are epidermal cells, more specifically, where the dermal cells are dermal fibroblasts and the epithelial cells are keratinocytes.

A micro-skin cell composite of the invention, wherein said mesenchymal cells are dermal cells and the epithelial cells are epidermal cells, more specifically, where the dermal cells are dermal fibroblasts and the epithelial cells are keratinocytes, has particular use in wound healing.

The inventors have identified rapid outgrowth from the micro-skin cell composites in *in vitro* cultivation, which confirms the clinical application of the composites in the treatment of wounds. The micro-skin cell composites of the invention are expected to provide improved survival and adherence to the wound bed, resulting in faster healing times and reduced scarring.

Alternatively, the micro-organ cell composite is a micro-follicular cell composite, wherein said mesenchymal cells are follicular dermal cells and the epithelial cells are epidermal cells, more specifically, where the dermal cells are follicular dermal cells and the epithelial cells are outer root sheath keratinocytes.

For example, micro-organ cell composite is a micro-follicular cell composite, wherein said mesenchymal cells are follicular dermal cells and the epithelial cells are epidermal cells, more specifically, where the dermal cells are follicular dermal cells and the epithelial cells are outer root sheath keratinocytes has particular use in follicular transplants and hair regeneration, i.e. in the treatment of alopecia is more common in men e. g., male pattern baldness, androgenic alopecia or female pattern baldness.

The inventors have identified that where suitable cell combinations are used (e.g. including hair follicle-derived cells), they can be employed for the creation of new hair follicle structures. Delivery is possible because micro-organ cell composites are small and easily gathered up and injected or placed in various parts of the body, to be delivered to anywhere and at any depth of skin. Conventional grafts, in contrast, would have to be placed on as a large sheet.

By replacing cells in the above-mentioned micro-organ cell composites with hair follicle epithelial cells; namely outer root sheath keratinocytes and follicular dermal cells, the microstructure can meet the basic criteria of hair biology-related organotypic systems according to Havlickova which includes 1) the two cell types are natively, physically interacting; 2) the structure contains basement membrane components; 3) epithelial cells show proliferation and keratinization and a low level of apoptosis; 4) dermal cells show minimal proliferation, minimal apoptosis and express specific hair follicle type secretory activities. Havlickova B, et al. Towards optimization of an organotypic assay system that imitates human hair follicle-like epithelial-mesenchymal interactions. Br J Dermatol. 151: 753-65 (2004); and Havlickova B, et al., A Human Folliculoid Microsphere Assay for Exploring Epithelial-Mesenchymal Interactions in the Human Hair Follicle. J Invest Dermatol. 129(4): 972-83 (2009). In meeting these basic criteria, the micro-organ cell composites of the present invention can serve as a good model to study hair induction *in vivo* or to provide a 3D *in vitro* screening system for management of hair growth disorder.

In one aspect, pharmaceutical compositions of the invention contain a therapeutically effective amount of a micro-organ cell composite in an amount suitable for administration to a patient, together with pharmaceutically-acceptable carrier. In one embodiment the composition further comprises one or more of the following: growth factors, lipids, genes, etc., or compounds for altering the acidity/alkalinity (pH) of the wound, or compounds for altering the growth and performance of the transplanted skin or hair cells and those at the margins of the wound.

The term "pharmaceutically-acceptable carrier" as used herein means one or more compatible solid or liquid fillers, diluents or encapsulating substances that are suitable for administration into a human. When administered, the pharmaceutical compositions of the present invention are administered in pharmaceutically acceptable preparations. Such preparations may routinely contain pharmaceutically acceptable concentrations of salt, buffering agents, preservatives, compatible carriers, cytokines and optionally other therapeutic agents, preferably agents for use in wound healing such as growth factors, peptides, proteolytic inhibitors, extracellular matrix components, steroids and cytokines. The term "carrier" denotes an organic or inorganic ingredient, natural or synthetic, with which the active ingredient is combined to facilitate the application. The term "pharmaceutically acceptable" means a non-toxic material that does not interfere with the effectiveness of the biological activity of the active ingredients. The term "physiologically acceptable" refers to a non-toxic material that is compatible with a biological system such as a cell, cell culture, tissue, or organism. As used herein, a pharmaceutically acceptable carrier includes any conventional carrier, such as those described in Remington's Pharmaceutical Sciences, by E. W. Martin, Mack Publishing Co, Easton, PA, 15th Edition (1975).

In a further aspect there is provided a micro-organ cell composite or a pharmaceutical composition in accordance with the invention for use as a medicament.

In a still further aspect the invention provides a micro-organ cell composite in accordance with the invention or pharmaceutical composition in accordance with the invention, for use in skin wound healing. Preferably, the micro-organ cell composite is a micro-skin cell composite, wherein said mesenchymal cells are dermal cells and said epithelial cells are epidermal cells, more specifically, where the dermal cells are dermal fibroblasts and the epithelial cells are keratinocytes.

In a still further aspect the invention provides a micro-organ cell composite in accordance with the invention or pharmaceutical composition in accordance with the invention, for use in follicular transplant and/or hair regeneration, i.e. for use in the treatment of alopecia e. g., male pattern baldness, androgenic alopecia or female pattern baldness. Preferably, the micro-organ cell composite is a micro-follicular cell composite, wherein said mesenchymal cells are follicular dermal cells and said epithelial cells are epidermal cells, more specifically, where the dermal cells are follicular dermal cells and the epithelial cells are outer root sheath keratinocytes.

Alternatively, the micro-organ cell composites of the invention can be used in tooth replacement or alternatively in organ replacement.

The compositions or micro-organ cell composites of the invention can be administered by any conventional route, including injection. The administration may, for example, be topical, intracavity, subcutaneous, or transdermal. Preferably the composition is prepared for topical administration.

The compositions or micro-organ cell composites of the invention are administered in effective amounts. An "effective amount" is the amount of a composition or micro-organ cell composites that alone, or together with further doses, produces the desired response. The compositions or micro-organ cell composites used in the foregoing methods preferably are sterile and contain an effective amount of the active ingredient for producing the desired response in a unit of weight or volume suitable for administration to a patient. The response can, for example, be measured by measuring the physiological effects of the composition or micro-organ cell composites upon the rate of or extent of wound healing or hair regeneration.

In a further aspect the invention provides a method for the treatment of skin or a skin wound, comprising applying to the skin, skin wound or skin wound bed a micro-skin cell composite of the invention or a pharmaceutical composition of the invention, wherein said mesenchymal cells are dermal cells and the epithelial cells are epidermal cells, more specifically, where the dermal cells are dermal fibroblasts and the epithelial cells are keratinocytes. The method is of particular use in skin re-epithelialisation. The term "re-epithelialisation" relates to the repair, replacement, functional recovery and ultimate regeneration of damaged epithelium inside the body (including skin), or outside the body. Alternatively, the method may be used in dermal replacemet.

As used herein the term wound relates to damaged tissues, preferably damaged skin, where the integrity of the skin or tissue is disrupted as a result from i.e. external force, bad health status, aging, exposure to sunlight, heat or chemical reaction or as a result from damage by internal physiological processes. Wounds where the epidermis is damaged are considered an open wound. Wound healing is the process of regenerating the covering cell layers of a tissue, preferably by re-epithelialisation or reconstruction.

In a further aspect the invention provides a method for follicular transplant and/or hair regeneration, or for the treatment of alopecia e. g., male pattern baldness, androgenic alopecia or female pattern baldness, comprising applying to a a micro-follicular cell composite of the invention or a pharmaceutical composition of the invention, wherein said mesenchymal cells are follicular dermal cells and the epithelial cells are epidermal cells, more specifically, where the dermal cells are follicular dermal cells and the epithelial cells are outer root sheath keratinocytes a micro-skin cell.

Preferably, said mesenchymal cells and/or said epithelial cells is/are autologous, i.e. said cells are derived from the individual to be treated or that biological material added to tissue cultures comes from the donor of the cells for tissue culture. Alternatively the cells may be non-autologous.

In a further aspect of the invention, the micro-organ cell composites of the invention provide an *in vitro* organ model, for example a model for understanding organ mesenchymal/epithelial interaction, a disease model for understanding disease progression, or a screening model.

A micro-organ cell composite according to the present invention can be utilised to clarify the complexity of epidermal/dermal interactions in a variety of settings. For instance, during the wound healing process, the crosstalk between the two types of cells plays a dominant role in a temporal and spatial manner by producing different profiles of growth factors, cytokines and genes, which gradually shift the microenvironment from an inflammatory to a synthesis-driven granulation tissue. The cell interactions also affect the contractile activity in dermal cells which is regarded as one of the characteristics of scar formation.

Accordingly, a micro-skin cell composite of the invention, wherein said mesenchymal cells are dermal cells and the epithelial cells are epidermal cells, more specifically, where the dermal cells are dermal fibroblasts and the epithelial cells are keratinocytes, can be used as a skin model. Alternatively, a micro-follicular cell composite, wherein said mesenchymal cells are follicular dermal cells and the epithelial cells are epidermal cells, more specifically, where the dermal cells are follicular dermal cells and the epithelial cells are outer root sheath keratinocytes, can be used as a follicular model.

A micro-skin or micro-follicle cell composite in accordance with the invention can be used as a skin model in culture work, for example for toxicity assays and in other applications. Advantageously, the micro-skin or micro-follicle cell composites of the present invention surprisingly do not require enzymatic treatment of cells before application, nor the involvement of biomaterials, thus optimising the quality of the applied cells and the potential for good recovery.

The micro-organ cell composites of the present invention requires low cell numbers and no extra substrate or chemicals; is easy and quick to produce; requires no complicated techniques; is repeatable with relatively large numbers capable of being made at any one time; and is less expensive than previously published 3D skin model systems. In addition, the composites are readily reproducible. Thus, the present micro-organ cell composites is suitable for investigating protein and gene expression of signalling molecules as well as global gene expression profiles under both normal and pathological conditions.

Further, such micro-organ cell composites can be used for preclinical research, pharmaceutical development, pharmaceutical and other skin testing, target gene therapy, toxicity testing, as a skin disease model, as a broader construct model for organs made from two cell types, for hair induction, and as a model for the study of epidermal and mesenchymal interactions. Preclinical strategies related to skin disease, tumour biology, scar formation etc., also benefit from micro-organ cell composites as described herein.

For certain applications, micro-organ cell composites may be formed by using cells from diseased tissues. For example, micro-skin cell composites can be formed using mesenchymal and / or epithelial cells from diseased skin tissue. Diseased skin tissue includes skin diseases such as cancers, skin wounds, and other skin disorders.

For example, abnormal micro-skin cell composite models which reflect clinical skin diseases can be established by using cells from clinical patients. The epithelium-mesenchymal interactions of micro-skin cell composite can then be compared with those of normal skin cells, which aids in studying the molecular and gene profile underlying the formation of diseases and in developing gene therapies.

Disease models using micro-skin cell composite can also be used in pharmaceutical testing or preclinical treatment for relevant skin diseases. Micro-skin cell composite provide an alternative and much improved tool to animal disease models and 2D cell culture systems.

After establishing the micro-skin cell composite structure from normal skin cells, which closely resemble the normal mesenchymal and epithelial cells interaction *in vivo,* cells from abnormal (e.g. diseased) tissue were studied to determine whether these cells can develop a similar structure and maintain the abnormal characteristics of the original abnormal tissue with the potential of establishing skin disease models without the use of animals. Interestingly, dermal cells derived from skin from patients with different diseases had variable capacity to support micro-skin cell composite formation.

In some of the examples below, keloid fibroblasts were used because the recent focus in the study of keloids has shifted from the role of the fibroblast (termed the "keloid fibroblast" when derived from keloids) to the epithelial-mesenchymal interactions in keloids. The former was thought to be primarily responsible for collagen and extracellular matrix (ECM) production which forms the bulk of keloid tissue. However, an increasing body of evidence has shown that autocrine, paracrine, and endocrine epithelial-mesenchymal interactions play a major role in not only normal skin homeostasis, growth, and differentiation but also scar contracture and scar pathogenesis. Lim IJ, et al. Fibroblasts cocultured with keloid keratinocytes: normal fibroblasts secrete collagen in a keloidlike manner. Am J Physiol Cell Physiol 283: C212-C222 (2002). (Conditioned medium from keloid keratinocyte/keloid fibroblast coculture induces contraction of fibroblast-populated collagen lattices.)

The observation of different expression patterns of basal membrane proteins of micro-skin cell composite of keloid fibroblasts from normal cells suggests that cells derived from keloid have at least kept some of their characteristics in micro-skin cell composite, if not all of them. Micro-skin cell composite comprising dermal cells and keratinocytes both derived from keloids would thus be an optimal *in vitro* model in which to investigate this interaction and manipulate cells behaviours, in order to find an effective treatment to keloid.

The micro-organ cell composites of the invention may be used to observe the effects of agents, e.g therapeutic agents, on organs, i.e. diseased organs, and to identify agents that are capable of treating diseases, and reducing the symptoms thereof. Preferably, the micro-organ is a micro-skin cell composite, wherein said mesenchymal cells are dermal cells and the epithelial cells are epidermal cells, more specifically, where the dermal cells are dermal fibroblasts and the epithelial cells are keratinocytes can be used as a skin model, still more specifically where said mesenchymal and / or epithelial cells are from diseased tissue.

Accordingly, the invention provides a method of screening compounds to identify agents useful for treating diseases, in particular skin diseases.

The method comprises providing a micro-skin cell composite, wherein said mesenchymal cells are dermal cells and the epithelial cells are epidermal cells, more specifically, where the dermal cells are dermal fibroblasts and the epithelial cells are keratinocytes, still more specifically where said mesenchymal and / or epithelial cells are from diseased tissue, contacting the micro-skin cell composite with a test compound, and determining the effect of the test compound on the micro-skin cell composite.

The test compounds are preferably administered to the micro-skin cell composite in an amount sufficient to and for a time necessary to exert an effect upon said micro-skin cell composite. These amounts and times may be determined by the skilled artisan by standard procedures known in the art.

The following examples illustrate embodiments of the invention, but should not be viewed as limiting the scope of the invention.

## Examples

### 1. Material and methods

Primary cell culture: healthy skin/hairy skin keloid tissue, haemangioma tissue from patients of both genders and various ages; fibroblasts from muscular dystrophy patients.

#### Keratinocyte cell culture from explants

Primary cultures of human keratinocytes were established. Skin samples were washed with double strength antibiotics (1.250 $\mu$g/ml amphotericin, 200 IU/ml penicillin and 200 $\mu$g/ml streptomycin) and small pieces of split thickness skin (about 2x2 mm) were obtained and transferred to the bottom of 35 mm diameter culture dishes (Primaria (Falcon) or Nunclon™ Surface (Denmark)) with a film of 1 ml of medium (consisting of MEM containing 20% foetal bovine serum (F7524, Sigma) and antibiotics (0.625ug/ml amphotericin B, 100 IU/ml penicillin and 100 $\mu$g/ml streptomycin) before cultivation at 37°C in 5% CO2 for 3 to 4 days.

Keratinocytes were then harvested from explants and subcultured in Epilife™ growth medium (Cascade Biologics) supplemented with human keratinocyte growth supplement (Cascade Biologics, 5 ml/500 ml). Cells were used within passages 4 to 5. Alternatively, keratinocytes were purchased from commercial suppliers (Promcell or Lonza).

#### Culture of dermal papilla cells

Skin samples were washed as described above. Primary cultures of dermal papilla cells were established from small pieces of full depth scalp skin as described in Randall VA, et al. Stem cell factor/c-Kit signalling in normal and androgenetic alopecia hair follicles. Journal of Endocrinology 197: 11-23 (2008).

Skin samples were micro-dissected in 100 mm sterile Petri dishes containing MEM medium, supplemented with glutamine (2 mmol/ml), penicillin (100 IU/ml), streptomycin (100 $\mu$g/ml) and amphotericin B (2.5 $\mu$g/ml); all supplements supplied by Gibco. Under a dissecting microscope (Leitz), each follicle was removed separately and individually microdissected to isolate the dermal papilla and surrounding sheath; each individual papilla/ sheath was then transferred to a 35 mm tissue culture-treated Petri dish (Bibby Sterilin, Stone, Staffordshire, UK) in the same medium supplemented with 20% serum.

8 to 10 dermal papillae were placed in one dish. Just prior to their introduction, the papillae were subjected to slight physical disruption as this was found to result in both a greater likelihood of attachment and earlier cellular activity. Jahoda C, Oliver RF. The growth of vibrissa dermal papilla cells in vitro. Br J Dermatol 105: 623-7 (1981).

Primary cultures were left untouched in a humidified incubator at 37 °C in 95% air: 5% $CO_2$ for more than a weeks to establish. Sufficient cells were harvested and subcultured in the same medium as above only with 10% FCS.

#### Primary culture of normal human skin fibroblasts, haemangioma fibroblasts, keloid fibroblasts and fibroblasts from muscular dystrophy patients

Fibroblasts were isolated from skin explants and expanded in conventional fibroblast medium The dermal tissue was washed with calcium and magnesium-free PBS, minced finely with sterile scissors, and allowed to adhere to tissue culture flasks for 30 min in an incubator at 37 °C in a humidified atmosphere of 5% $CO_2$ in MEM supplied with 20% FCS. Fibroblasts were subcultured and maintained under the same conditions as hair follicle dermal cells.

## 2. Preparation of micro-skin cell composite

Example 1: Formation of dermal cell aggregates

Dermal cell aggregations were formed using the hanging droplet method as described in Kurosawa H. Methods for inducing embryoid body formation: in vitro differentiation system of embryonic stem cells. J. Biosci. Bioeng 103: 389-398 (2007). Single dermal cell suspension was achieved in MEM supplied with 10% FCS. 3000 dermal cells/10$\mu$l were applied on the lid of a 100-mm Petri dish. The lid was then inverted and placed over the bottom of a Petri dish filled with PBS to prevent the drops from drying out. A further 100-mm dish filled with PBS was placed on top of the Petri dish containing hanging drops to generate a sustained pressure to the droplets. When the lid is inverted, each drop hangs and the dermal cells travel to the bottom of the drop. The hanging droplets were then returned to a 37°C, 5% $CO_2$ incubator for a further two days to allow the single cells to form an aggregate ball structure.

Example 2: Application of epidermal cells

Cultured primary human keratinocytes were used within passage 5. About 70% confluent keratinocytes were dissociated with 0.25% trypsin-EDTA, and neutralized with 10 % FCS MEM. Cells were spun down and re-suspended into single cell suspension in Epilife™ growth medium. 3000 cells /10ul were added to each hanging droplet bearing a dermal cell aggregation. The mixture culture was then returned to a 37°C, 5% $CO_2$ incubator for a further two days to achieve the micro-skin structure. Alternatively, HaCaT cells were used as a control.

Maintenance of micro-skin cell composite in culture

For longer term cultivation, the micro-skin cell composite was carefully transferred into 20 $\mu$l fresh medium composed of 10% FCS MEM and Epilife with 1:1 ratio.

Re-growth of micro-skin cell composite structure

3 days after the formation of the micro-skin cell composite, the cells were transferred to a 36 well Petri dish with normal culture medium (MEM with 10% FCS) for a further cultivation to check the capability of cell outgrowth from the skin structure.

Histology

For morphological analysis of the micro-skin cell composite, samples were embedded in OCT embedding compound, and snap frozen in liquid nitrogen. Sections (10 $\mu$m) were then cut, and, after fixation in ice-cold acetone, the samples were stained with hematoxylin-eosin (Sigma).

Immuno fluorescence

Cryo-sections were fixed with acetone for 10 minutes, and again washed in PBS before blocking with 10% donkey serum (D9663, Sigma) in PBS. Sections were then incubated with primary antibodies overnight at 4°C followed by Alexa-Red or FITC conjugated secondary antibodies with 4'-6-Diamidino-2-phenylindole (DAPI) at room temperature for 2 hours. The slides were then washed and mounted.

The primary antibodies included: suprabasal and differentiating keratinocyte marker K10 (mouse anti-cytokeratin 10 monoclonal antibody, Chemicon International); keratin15 (mouse monoclonal keratin 15 antibody, Lab vision); CD34 (mouse monoclonal to CD34, abcam); P63 (mouse monoclonal, clone 4A4, Labvision); filaggrin, involucrin, fibronectin, Laminin, vimentin, collagen VII. The secondary antibodies used were Alexa Fluor 594 donkey antimouse IgG, and Alexa Fluor 488 donkey anti-goat IgG (both from Invitrogen). Cells and tissue samples were then examined and images obtained using a Zeiss (Axio Imager.M1, Germany) fluorescence microscope (or Zeiss LSM 510 Confocal microscope from Carl Zeiss, Germany), with an Openlab imaging system (Improvision).

Results

Characteristics of epithelial and mesenchymal cells interaction in micro-skin cell composites The formation of micro-skin cell composites

Mesenchymal cells aggregation initially took place within 24 hours. It reached a smooth round sphere structure in 2 to 3 days. A mixture of epidermal and mesenchymal spheroid cell aggregates was achieved one day after the application of keratinocytes onto the dermal sphere with dermal cells in the centre of the structure embraced by epithelial cells as the outer layer. Such structures were taken for histology and IF study on 6, 9 and 14 days after micro-skin cell composite formation.

Cell viability in micro-skin cell composites

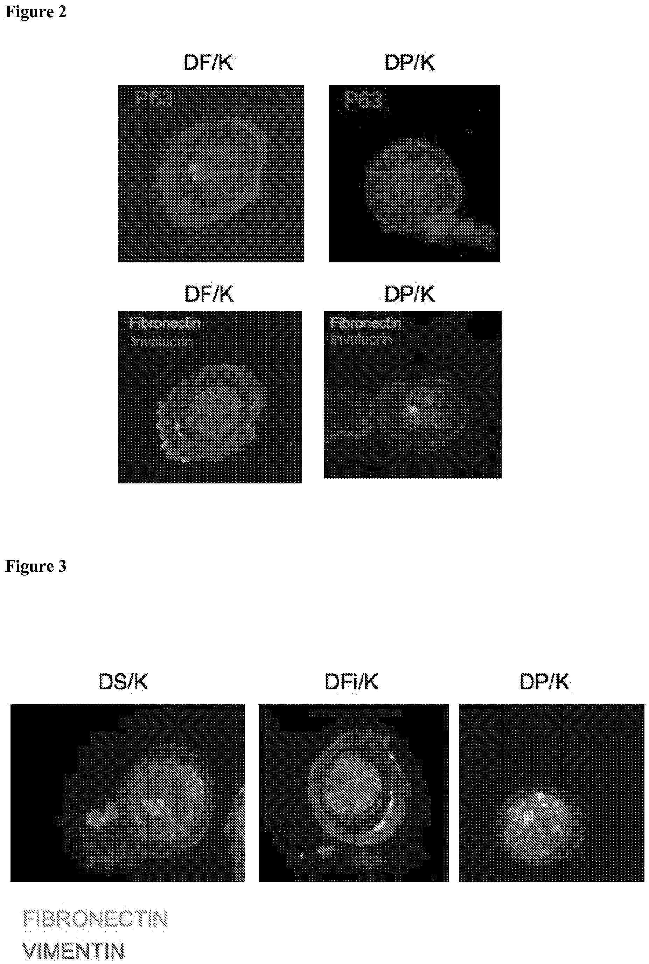

Three types of dermal cells were employed in this study: interfollicular dermal fibroblasts, hair follicle dermal papilla, and dermal sheath cells. The aggregate of mesenchymal dermal cells was in close contact with keratinocytes in a distinct skin structural manner (see Figure 2). The viability of cells in the micro-skin cell composites at three time points was investigated. Within the observation period of 14 days, there were few signs of apoptosis with nucleic fragmentation (DAPI labelling Figure 2). Interestingly, positive expressing P63 and CD34 cells which normally appear on the basal layer of human skin epidermis and are regarded as epidermal stem cell markers were found in the cell layer above the basement membrane of the cultured micro-skin cell composites (Figure 2). In spheres formed with dermal cells alone which were cultured as a control group cell death/ necrosis started from the centre from about 6 days.

Micro-skin cell composites exhibited close epithelial/mesenchymal interaction and tissue specific labelling

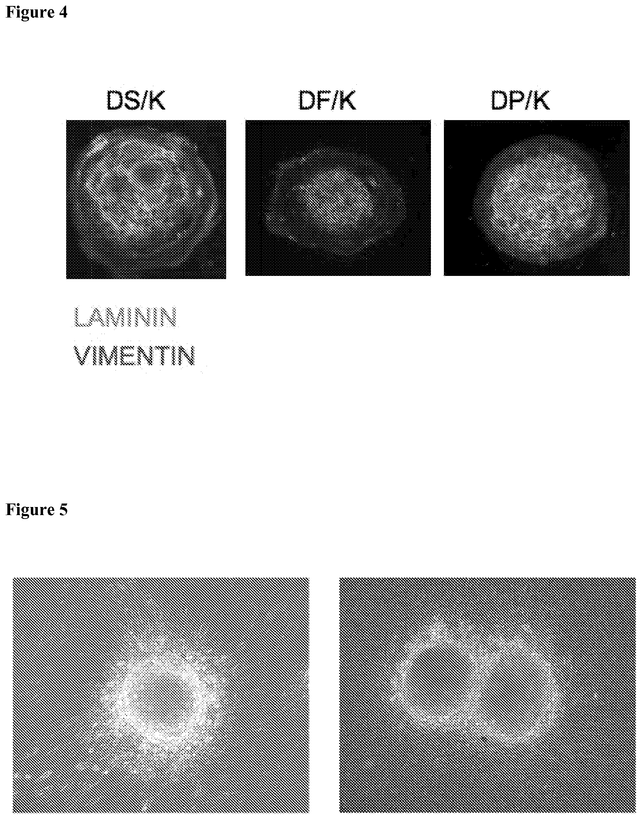

The epidermal layer and dermal cell aggregations were in close physical contact through a basement membrane which was identified by basement membrane components such as collagen VII. In addition, epithelial cells expressed early differentiating marker cytokeratin 10 in the middle layer of the epidermis area, and increasingly expressed differentiation markers, involucrin and filaggrin, in the epidermal layers (Figure 3). At the same time dermal cells strongly expressed extracellular matrix constituents including fibronectin (Figures 3 and 4) the mesenchymal cell marker vimentin was exclusively expressed in the core dermal cell aggregate (Figure 4). Not all cell combinations exhibited exactly the same expression. Within the micro-organs created from hair follicle dermal sheath/keratinocyte and dermal papilla/keratinocyte combinations, prominent acellular regions that showed high levels of expression of extracellular matrix components developed over time (Figures 3 and 4). These were interpreted as being secreted pools of extracellular matrix.

Capacity of micro-skin cell composites to reproduce outgrowth when returned to normal culture condition

After 5 to 7 days, the cells returned to normal cultural condition (MEM supplied with 10% FCS), cells with dermal morphology migrated out of micro-skin cell composites (from both attached and floating micro-skin cell composites), and were able to replicate to confluence (Figure 5). In addition, cells have shown signs of aggregation.

Ability of micro-skin cell composites to recover from deep frozen storage

Micro-skin cell composites were defrosted and cultured in MEM containing 10% FCS after being deep frozen in DMSO/FCS in liquid nitrogen without disturbance. Dermal cells were seen to migrate out from micro-skin cell composites about 1 week after being defrosted as the MSEs were attached to the bottom of the culture dish. The outgrowths presented in a similar manner as they were before having been frozen.

Plasticity of micro-skin cell composites

With a replacement of skin dermal cells, micro-skin cell composite structures were also achieved by using mesenchymal cells cultured from keloid and haemangioma in conjunction with HaCaT keratinocytes. Morphologically, these micro-skin cell composites appeared no different from those made from normal skin cells. However, immunofluorescence revealed some differences of marker expression from diseased cells. For instance, there was an absence of collagen VII expression and less organized laminin expression from micro-skin cell composites composed by keloid fibroblasts and HaCaT cells, whereas in the micro-organs with core haemangioma dermal cells type VII collagen was highly expressed (Figure 6).

**3. Confirmation of micro-organ size and geometry**

Formation of micro-skin cell composites

Dermal spheres where generated as described by Higgins et al 2010, briefly dermal cells were grown in T25 flasks with 5ml MEM (Invitrogen) with 10% foetal calf serum (Biosera) and penicillin/streptomycin in a 37°C incubator with $CO_2$ at 5%. Vigorously growing cultures gave cleaner spheres, thus 24hrs before use near confluent flasks are passaged with trypsinisation and 9/10th of the culture seeded into a clean T25 flask with MEM 10% FCS from this point on the removal of antibiotic from the grow media aided sphere formation. For spherical micro-skin cell composites the cells were removed from the flask with trypsin, spun at 1000rpm and re suspended in 1 ml MEM 10% FCS. Of this suspension 10μl were mixed with 10μl trypan blue and viable cells counted. The cell suspension would be diluted to give an appropriate number of cells per μl and 10μl drops were placed on to the lid of bacteriological dish in a 10x10mm grid. The dish lid was inverted and the dish base filled with 25ml sterile distilled water. The cells were then incubated for four days before addition of epidermal cells, the dermal cells would usually have aggregated into a clean sphere by 24-48hrs.

Measuring micro-skin cell composites

Micro-skin cell composites were photographed using a low power microscope (5x magnification) and sphere sizes measured using ImageJ software (http://rsbweb.nih.gov/ij/). Spheres of 3000-1000 cells formed in 10μl drops, spheres of 600 cells and fewer formed in 5μl drops.

Epidermal coating dermal spheres

The epidermal NHEK cells (Promocell) were grown in T25 flasks in KGM2 media (Promocell) with appropriate supplements in a 37°C incubator with CO2 at 5%. Cells which were close to confluence were removed from the flask with trypsin spun at 1000rpm and re suspended in 1 ml KGM2. Of this suspension 10μl were mixed with 10μl trypan blue and viable cells counted. The cell suspension would be diluted to give and appropriate number of cells per μl and 10μl were added to each dermal sphere drop. Great care must be taken when turning the bacteriological plate lid with the droplets when working with 20μl drops. The dermal spheres with epidermal cells were incubated and observed for a further 4 days, after this time the epidermal cells would have aggregated around the dermal sphere to give an even coating.

Epidermal aggregation

To investigate the aggregation of epidermal cells alone, epidermal cells were resuspended in KGM2 and viable cells counted. The cell suspension was diluted with KGM2 to give 600 cells μl-1. The cell suspension was then divided and an equal volume of MEM containing foetal calf serum dilutions was added to give an end serum concentration range of 1% through to 5%. This was used to give five drops of each serum concentration.

Results

Micro-skin cell composites size predictable by simple geometry

The formation of micro-skin cell composite spheres follows simple geometry. If we make a dermal sphere with 3000 cells and assume each cell has a volume of 1 cubic unit then the sphere has a volume of 3000U^3. The radius of a sphere can be found from: V=4/3πr^3

$$r = \sqrt[3]{(V/(4/3))/\pi}$$

So for a 3000U^3 sphere the radius is 8.95U thus diameter is 17.9U, this means our cell aggregate will be approximately 18 cells across.

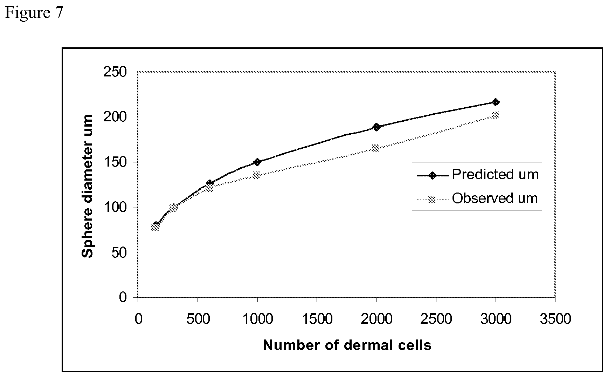

The average measurement of five 3000 cells dermal spheres was 216.5um, dividing this by the number of cells means the diameter of a cell and the size of one unit is 12.1um. With the size of a single cell in an aggregate known, sizes of any dermal sphere can be predicted and compared to real spheres, as illustrated in figure 7 and table 1 below.

| Dermal cells | Predicted um | Observed um |
| --- | --- | --- |
| 150 | 79.7 | 77.8 |
| 300 | 100.5 | 98.7 |
| 600 | 126.6 | 121.4 |
| 1000 | 150.1 | 134.7 |
| 2000 | 189.1 | 165.1 |
| 3000 | 216.5 | 201.1 |

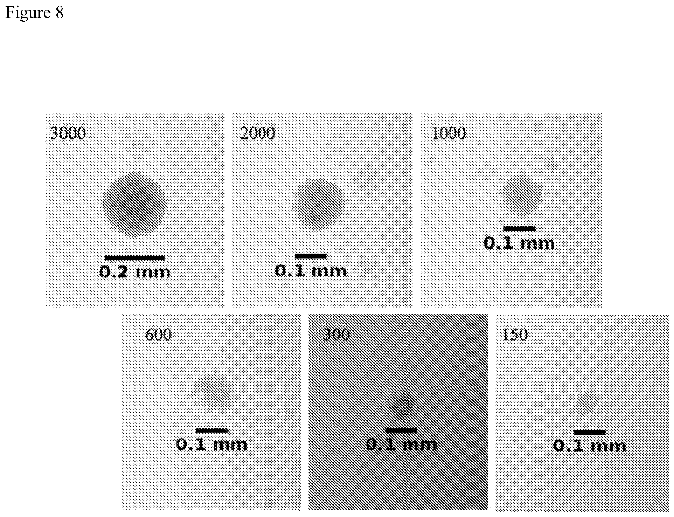

The slight variation between predicted and observed sizes are likely due a small percentage of cells in each drop not being incorporated into the sphere, however the difference between the predicted and observed sizes for the 3000 cells sphere (and the observed sizes in two different runs) is 15um which is approximately the width of one cell, as illustrated in figure 8.

4. Epidermal coating aggregation is active

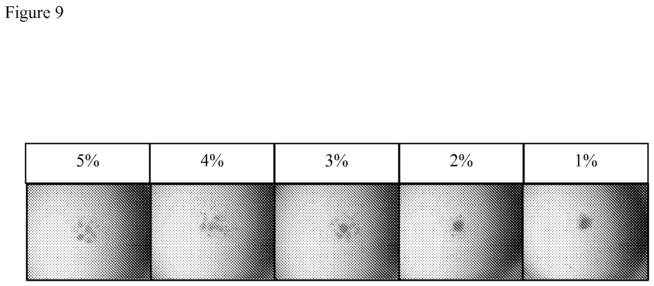

The formation of both dermal and epidermal sphere is probably an active aggregation on the part of the cell and not due to gravity/physical forces. Were the cells to be forming spheres due solely to gravity we would expect dermal and epidermal cells aggregate regardless of media composition. Figure 9 shows the aggregation of epidermal cells in 1:1 KGM2:MEM media with different foetal calf serum concentrations.

The serum in the media will influence a great many cellular processes however it would not have any effect on a purely physical process such as gravity.

Discussion

The aggregation of dermal and epidermal cells into three dimensional structures potentially provides a versatile model for whole skin which lends it's self to automated setup and high though put screening. As the dermal cells pack together 'perfectly' the size of cell aggregate spheres can be accurately predicted. Coupled with this the formation of cellular aggregates is an active process on the part of the cells, this means that the aggregation of cells can be used to compare different cell types, cells from healthy or diseased tissues and the effects of agents added to the media. With known sizes, a poorly aggregating cell type will be apparent due to the reduced sphere diameter, increased aggregation time or reduced ability to recruit and support another cell type to the sphere. The epidermal cell aggregation happens quite readily with 1% serum but with 5% serum the epidermal cells do little more than form loose clumps. Thus the epidermal coating of dermal spheres at 5% serum is due to active aggregation and testament to the supporting role dermal cells can play to epidermal cells.

Micro-organ cell composite sphere formation is an active process

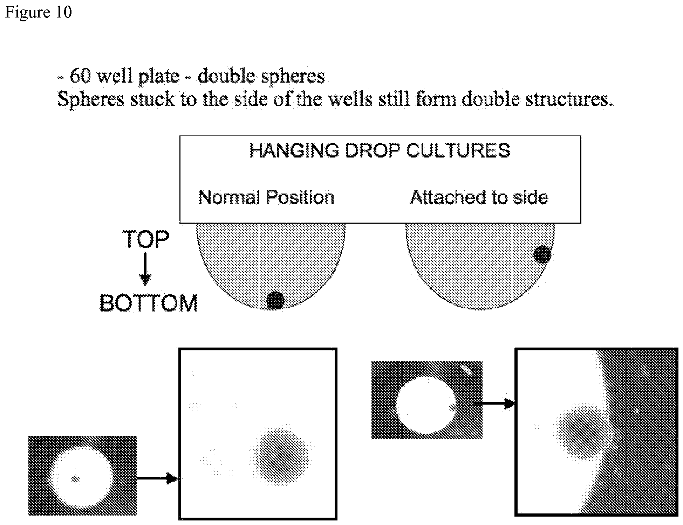

On occasions dermal spheres stick to the sides of the hanging drops when the epidermal cells are added. These still form double layered, mesenchymal /epithelial micro organ cell composites, as outline in figure 10. Were this a "gravity only" process, it would be predicted that the epidermal cells would form separate spheres at the base of the drops rather than make double structures on top of the dermal balls.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive.

**Claims**

1. A micro-organ composite which comprises a core group of cells and an outer layer of cells, wherein the core group of cells is fully encapsulated by the outer layer of cells and wherein:

   (a) the cells of the core group are mesenchymal cells and the cells of the outer layer are epithelial cells; or
   (b) the cells of the core group are epithelial cells and the cells of the outer layer are mesenchymal cells;
   wherein said core group of cells and said outer layer of cells interact so as to form a basement membrane.

2. The composite according to claim 1, wherein:

   a) the mesenchymal cells and/or the epithelial cells are derived from more than one cell source; and/or
   b) the mesenchymal cells are dermal cells, and wherein the epithelial cells are epidermal cells, optionally wherein said epidermal cells are keratinocytes; and/or
   c) the composite is in the form of a spherical particulate, optionally wherein the spherical particulate has a size between 40 and 500 microns.

3. The composite according to any one of the preceding claims for use as a medicament.

4. The composite according to any one of the claims 1 or 2 for use in skin wound healing, optionally wherein the dermal cells are dermal fibroblasts and the epithelial cells are keratinocytes.

5. The composite according to any one of claims 1 to 2 for use in treating alopecia, optionally wherein the dermal cells are follicular dermal cells and the epithelial cells are outer root sheath keratinocytes.

6. A pharmaceutical composition comprising the composite according to any one of claims 1 to 2 together with a pharmaceutically acceptable carrier.

7. The composite for use according to any one of claims 4 to 5 or the pharmaceutical composition according to claim 6, wherein said composite or composition is prepared for topical administration.

8. A method of producing a micro-organ cell composite, which comprises a core group of cells and an outer layer of cells, wherein the core group of cells is fully encapsulated by the outer layer of cells, said method comprising:

   (i)

      a) growing disaggregated mesenchymal cells in a hanging drop culture to form an aggregate core of cells; and
      b) adding epithelial cells to the aggregate core of cells,

   wherein the epithelial cells grow to form an outer layer on the aggregate core of cells; or
   (ii)

      a) growing disaggregated epithelial cells in a hanging drop culture to form an aggregate core of cells; and
      b) adding mesenchymal cells to the aggregate core of cells,

   wherein the mesenchymal cells grow to form an outer layer on the aggregate core of cells.

9. The method according to claim 8, wherein:

   a) the mesenchymal cells are dermal cells, and wherein the epithelial cells are epidermal cells, optionally wherein said epidermal cells are keratinocytes; or
   b) the aggregate core of cells and the outer layer establish a basement membrane, preferably in less than 2 days, less than 3 days, less than 4 days, less than 5 days or less than 6 days.

10. The method of according to any one of claims 8 or 9, wherein the cells of the composite are viable for about 2 weeks or more, or about 3 weeks or more.

11. An in vitro model for studying a skin disease or disorder comprising the composite according to any one of claims 1 to 2.

12. Use of a composite according to any one of claims 1 to 2 as an in vitro skin model, optionally wherein said skin model is a skin disease or disorder model.

13. A method of screening an agent for the treatment of a skin disease or disorder comprising:

    a) providing a composite according to any one of claims 1 to 2;
    b) exposing said composite to an agent; and
    c) determining whether the agent has a therapeutic effect on the composite.

14. The in vitro model according to claim 11, the use according to claim 12 or the method according to claim 13, wherein the skin disease or disorder is a keloid, a tumour, or a wound.

15. A method of screening for any molecular or chemical agent:

    a) providing a composite according to any one of claims 1 to 2;
    b) exposing said composite to an agent; and
    c) determining whether the agent has a toxic effect on the composite, or affects cell growth or viability, or alters gene expression in the composite cells.


**Patentansprüche**

1. Ein Mikroorgan- Verbund, der eine Kerngruppe von Zellen und eine äußere Schicht aus Zellen umfasst, wobei die Kerngruppe von Zellen vollständig von der äußeren Schicht von Zellen eingekapselt ist und wobei:

    (a) die Zellen der Kerngruppe mesenchymale Zellen sind und die Zellen der äußeren Schicht epitheliale Zellen sind; oder
    (b) die Zellen der Kerngruppe epitheliale Zellen sind und die Zellen der äußeren Schicht mesenchymale Zellen sind;
    wobei jene Kerngruppe von Zellen und jene äußere Schicht von Zellen so interagieren, dass sie eine Basalmembran bilden.

2. Der Verbund nach Anspruch 1, wobei:

    a) die mesenchymalen Zellen und/oder die epithelialen Zellen von mehr als einer Zellquelle abgeleitet sind; und/oder
    b) die mesenchymalen Zellen dermale Zellen sind, und wobei jene epithelialen Zellen epidermale Zellen sind, wobei die epidermalen Zellen gegebenenfalls Keratinozyten sind; und/oder
    c) der Verbund in Form eines kugelförmigen Teilchens vorliegt, wobei gegebenenfalls das kugelförmige Teilchen eine Größe zwischen 40 und 500 Mikron hat.

3. Der Verbund nach einem der vorhergehenden Ansprüche zur Verwendung als Medikament.

4. Der Verbund nach einem der Ansprüche 1 oder 2 zur Verwendung bei der Heilung von Hautwunden, wobei die dermalen Zellen gegebenenfalls dermale Fibroblasten sind und die epithelialen Zellen Keratinozyten sind.

5. Der Verbund nach einem der Ansprüche 1 bis 2 zur Verwendung bei der Behandlung von Alopezie, gegebenenfalls wobei die dermalen Zellen follikuläre dermale Zellen sind und die epithelialen Zellen Keratinozyten der äußeren Wurzelscheide sind.

6. Eine pharmazeutische Zusammensetzung, umfassend: den Verbund nach einem der Ansprüche 1 bis 2 zusammen mit einem pharmazeutisch akzeptablen Träger.

7. Der Verbund zur Verwendung nach einem der Ansprüche 4 bis 5 oder die pharmazeutische Zusammensetzung nach Anspruch 6, wobei jener Verbund oder die Zusammensetzung für die topische Verabreichung hergestellt ist.

8. Ein Verfahren zur Herstellung eines Mikroorgan-Zellverbundes, der eine Kerngruppe von Zellen und eine äußere Schicht von Zellen umfasst, wobei die Kerngruppe von Zellen vollständig von der äußeren Schicht von Zellen eingekapselt ist, wobei jenes Verfahren umfasst:

    (i)

        a) Wachsenlassen disaggregierter mesenchymaler Zellen in einer hängenden Tropfenkultur, um einen aggregierten Zellkern zu bilden; und
        b) Hinzufügen von epithelialen Zellen zum aggregierten Zellkern,
        wobei die epithelialen Zellen wachsen, um eine äußere Schicht auf dem aggregierten Kern von Zellen zu bilden; oder

    (ii)

        a) Wachsenlassen disaggregierter epithelialen Zellen in einer hängenden Tropfenkultur, um einen aggregierten Zellkern zu bilden; und
        b) Hinzufügen mesenchymaler Zellen zum aggregierten Zellkern,
wobei die mesenchymalen Zellen wachsen, um eine äußere Schicht auf dem aggregierten Kern von Zellen zu bilden.

**9.** Das Verfahren nach Anspruch 8, wobei:

a) die mesenchymalen Zellen dermale Zellen sind, und wobei die epithelialen Zellen epidermale Zellen sind, wobei jene epidermalen Zellen gegebenenfalls Keratinozyten sind; oder
b) der aggregierte Zellkern und die äußere Schicht eine Basalmembran bilden, vorzugsweise in weniger als 2 Tagen, weniger als 3 Tagen, weniger als 4 Tagen, weniger als 5 Tagen oder weniger als 6 Tagen.

**10.** Das Verfahren nach einem der Ansprüche 8 oder 9, wobei die Zellen des Verbundes etwa 2 Wochen oder länger, oder etwa 3 Wochen oder länger lebensfähig sind.

**11.** Ein In-vitro-Modell zur Untersuchung einer Hautkrankheit oder -störung, umfassend den Verbund nach einem der Ansprüche 1 bis 2.

**12.** Verwendung eines Verbundes nach einem der Ansprüche 1 bis 2 als In-vitro-Hautmodell, wobei gegebenenfalls jenes Hautmodell ein Hautkrankheitsoder - störungsmodell ist.

**13.** Ein Verfahren zum Screening eines Agens für die Behandlung einer Hautkrankheit oder - störung, umfassend:

a) Bereitstellung eine Verbundes nach einem der Ansprüche 1 bis 2;
b) Aussetzen jenes Verbundes einem Agens; und
c) Bestimmung, ob das Agens eine therapeutische Wirkung auf den Verbund hat.

**14.** Das In-vitro-Modell nach Anspruch 11, die Verwendung nach Anspruch 12 oder das Verfahren nach Anspruch 13, wobei die Hautkrankheit oder -störung ein Keloid, ein Tumor oder eine Wunde ist.

**15.** Ein Verfahren zum Screening auf molekulare oder chemische Agentien:

a) Bereitstellung eines Verbundes nach einem der Ansprüche 1 bis 2;
b) Aussetzen jenes Verbundes einem Agens; und
c) Bestimmung, ob das Agens eine toxische Wirkung auf den Verbund hat oder das Zellwachstum oder die Lebensfähigkeit beeinflusst, oder die Genexpression in den Verbund-Zellen verändert.

**Revendications**

**1.** Composite micro-organique comprenant un groupe central de cellules et une couche externe de cellules, dans lequel le groupe central de cellules est entièrement encapsulé par la couche externe de cellules et dans lequel :

(a) les cellules du groupe central de cellules sont des cellules mésenchymateuses et les cellules de la couche externe sont des cellules épithéliales ; ou
(b) les cellules du groupe central de cellules sont des cellules épithéliales et les cellules de la couche externe sont des cellules mésenchymateuses ;
dans lequel ledit groupe central de cellules et ladite couche externe de cellules interagissent de manière à former une membrane basale.

**2.** Composite selon la revendication 1, dans lequel :

a) les cellules mésenchymateuses et/ou les cellules épithéliales sont dérivées de plus d'une source cellulaire ; et/ou
b) les cellules mésenchymateuses sont des cellules dermiques, et dans lequel les cellules épithéliales sont des cellules épidermiques, facultativement dans lequel lesdites cellules épidermiques sont des kératinocytes ; et/ou
c) le composite est sous la forme d'une particule sphérique, facultativement dans lequel la particule sphérique a une taille comprise entre 40 et 500 microns.

**3.** Composite selon l'une quelconque des revendications précédentes pour une utilisation comme médicament.

**4.** Composite selon l'une quelconque des revendications 1 ou 2 pour une utilisation dans la cicatrisation des plaies cutanées, facultativement dans lequel les cellules dermiques sont des fibroblastes dermiques et les cellules épithéliales sont des kératinocytes.

**5.** Composite selon l'une quelconque des revendications 1 à 2 destiné à être utilisé dans le traitement de l'alopécie,
facultativement dans lequel les cellules dermiques sont des cellules dermiques folliculaires et les cellules épithéliales sont des kératinocytes de la gaine épithéliale externe.

**6.** Composition pharmaceutique comprenant le composite selon l'une quelconque des revendications 1 à 2 conjointement à un support pharmaceutiquement acceptable.

7. Composite pour une utilisation selon l'une quelconque des revendications 4 à 5 ou composition pharmaceutique selon la revendication 6, dans lequel ledit composite ou ladite composition est préparé(e) pour une administration topique.

8. Procédé de production d'un composite cellulaire micro-organique, comprenant un groupe central de cellules et une couche externe de cellules, dans lequel le groupe central de cellules est entièrement encapsulé par la couche externe de cellules, ledit procédé comprenant :

(i)

a) la croissance de cellules mésenchymateuses désagrégées dans une culture en goutte suspendue pour former un agrégat central de cellules ; et
b) l'ajout de cellules épithéliales à l'agrégat central de cellules,

dans lequel les cellules épithéliales se développent pour former une couche externe sur l'agrégat central de cellules ; ou
(ii)

a) la croissance de cellules épithéliales désagrégées dans une culture en goutte suspendue pour former un agrégat central de cellules ; et
b) l'ajout de cellules mésenchymateuses à l'agrégat central de cellules,

dans lequel les cellules mésenchymateuses se développent pour former une couche externe sur l'agrégat central de cellules.

9. Procédé selon la revendication 8, dans lequel :

a) les cellules mésenchymateuses sont des cellules dermiques, et dans lequel les cellules épithéliales sont des cellules épidermiques, facultativement dans lequel lesdites cellules épidermiques sont des kératinocytes ; ou
b) l'agrégat central de cellules et la couche externe établissent une membrane basale, de préférence en moins de 2 jours, moins de 3 jours, moins de 4 jours, moins de 5 jours ou moins de 6 jours.

10. Procédé selon l'une quelconque des revendications 8 ou 9, dans lequel les cellules du composite sont viables pendant environ 2 semaines ou plus, ou environ 3 semaines ou plus.

11. Modèle in vitro pour étudier une maladie de peau ou un trouble cutané comprenant le composite selon l'une quelconque des revendications 1 à 2.

12. Utilisation d'un composite selon l'une quelconque des revendications 1 à 2 comme modèle cutané in vitro, facultativement dans laquelle ledit modèle cutané est un modèle de maladie de peau ou de trouble cutané.

13. Procédé de criblage d'un agent pour le traitement d'une maladie de peau ou d'un trouble cutané comprenant :

a) la fourniture d'un composite selon l'une quelconque des revendications 1 à 2 ;
b) l'exposition dudit composite à un agent ; et
c) la détermination de savoir si l'agent a un effet thérapeutique sur le composite.

14. Modèle in vitro selon la revendication 11, utilisation selon la revendication 12 ou procédé selon la revendication 13, dans lequel ou laqulle la maladie de peau ou le trouble cutané est une chéloïde, une tumeur ou une plaie.

15. Procédé de criblage de tout agent moléculaire ou chimique comprenant :

a) la fourniture d'un composite selon l'une quelconque des revendications 1 à 2 ;
b) l'exposition dudit composite à un agent ; et
c) la détermination de savoir si l'agent a un effet toxique sur le composite, ou affecte la croissance ou la viabilité cellulaire, ou modifie l'expression génique dans les cellules composites.

**Figure 1**

**Figure 2**

DF/K                   DP/K

DF/K                   DP/K

**Figure 3**

DS/K            DFi/K           DP/K

FIBRONECTIN
VIMENTIN

**Figure 4**

DS/K　　　DF/K　　　DP/K

LAMININ
VIMENTIN

**Figure 5**

**Figure 6**

Haemangioma    Normal Fi    Muscular Dystrophy    Keloid

Collagen VII
Filaggrin

Haemangioma/K    Normal abdominal/K

Figure 7

Figure 8

Figure 9

| 5% | 4% | 3% | 2% | 1% |
|----|----|----|----|----|

Figure 10

- 60 well plate - double spheres
Spheres stuck to the side of the wells still form double structures.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5888248 A
- US 5945101 A
- WO 9116010 A
- WO 03041568 A
- EP 2034011 A
- EP 1734113 A
- WO 2007100870 A

### Non-patent literature cited in the description

- **MEDAWAR, PB.** Sheets of pure epidermal epithelium from human skin. *Nature,* 1941, vol. 148, 783-4
- **METCALFE AD ; FERGUSON MW.** *Biomaterials,* December 2007, vol. 28 (34), 5100-13
- **C M YEN ; C C CHAN ; S J LIN.** *Biomaterials,* 2010, vol. 31 (15), 4341-4352
- **GANGATIRKAR, P. et al.** Establishment of 3D organotypic cultures using human neonatal epidermal cells. *Nat. Protoc.,* 2007, vol. 2, 178-86
- **HAVLICKOVA B et al.** Towards optimization of an organotypic assay system that imitates human hair follicle-like epithelial-mesenchymal interactions. *Br J Dermatol.,* 2004, vol. 151, 753-65
- **HAVLICKOVA B et al.** A Human Folliculoid Microsphere Assay for Exploring Epithelial-Mesenchymal Interactions in the Human Hair Follicle. *J Invest Dermatol.,* 2009, vol. 129 (4), 972-83
- **E. W. MARTIN.** Remington's Pharmaceutical Sciences. Mack Publishing Co, 1975
- **LIM IJ et al.** Fibroblasts cocultured with keloid keratinocytes: normal fibroblasts secrete collagen in a keloidlike manner. *Am J Physiol Cell Physiol,* 2002, vol. 283, C212-C222
- **RANDALL VA et al.** Stem cell factor/c-Kit signalling in normal and androgenetic alopecia hair follicles. *Journal of Endocrinology,* 2008, vol. 197, 11-23
- **JAHODA C ; OLIVER RF.** The growth of vibrissa dermal papilla cells in vitro. *Br J Dermatol,* 1981, vol. 105, 623-7
- **KUROSAWA H.** Methods for inducing embryoid body formation: in vitro differentiation system of embryonic stem cells. *J. Biosci. Bioeng,* 2007, vol. 103, 389-398